# EUROPEAN PATENT APPLICATION

(11) **EP 4 104 855 A1**
(43) Date of publication of application: **21.12.2022**
(21) Application number: 21753156.5
(22) Date of filing: 10.02.2021
(51) Int. Cl.: A61K 39/395, C07K 16/28, A61P 35/00

(54) **USE OF ANTI-PD-1 ANTIBODY IN TREATING NEUROENDOCRINE TUMORS**

(30) Priority: 13.02.2020 CN 202010090965
(71) Applicant: Shanghai Junshi Biosciences Co., Ltd., Pilot Free Trade Zone Shanghai 201210 (CN)
(72) Inventor: YAO, Sheng, Shanghai 201210 (CN); FENG, Hui, Shanghai 201210 (CN); WU, Hai, Shanghai 201210 (CN)
(74) Representative: V.O.
(86) International application number: PCT/CN2021/076458
(87) International publication number: WO 2021/160152

(57) **Abstract**

The present invention relates to use of an anti-PD-1 antibody and/or an antigen-binding fragment thereof in the treatment of a neuroendocrine neoplasm. The present invention also relates to an agent or a kit for detecting an *ARID1A* gene mutation or amplification, or a chromosomal gene rearrangement, and use of the detection agent or kit in predicting the therapeutic effect of an anti-PD-1 antibody or an antigen-binding fragment thereof in the treatment of a patient with a neuroendocrine neoplasm.

## Description

### TECHNICAL FIELD

The present invention relates to use of an anti-PD-1 antibody or an antigen-binding fragment thereof in the treatment of a neuroendocrine neoplasm; and use of an anti-PD-1 antibody or an antigen-binding fragment thereof in the preparation of a medicament for treating a neuroendocrine neoplasm; and a method for predicting the therapeutic effect of an anti-PD-1 antibody or an antigen-binding fragment thereof on a neuroendocrine neoplasm using a biomarker.

### BACKGROUND

Immune escape is one of the characteristics of cancer. Ahmadzadeh, M. et al disclosed in Blood, 114: 1537-44 that tumor-specific T lymphocytes are often present in the tumor microenvironment, draining lymph nodes and peripheral blood, but are generally unable to control tumor progression due to the network of immunosuppressive mechanisms present in the tumor microenvironment. CD8⁺ tumor infiltrating T lymphocytes (TILs) generally express activation-induced inhibitory receptors, including CTLA-4 and PD-1, while tumor cells often express immunosuppressive ligands, including PD-1 ligand 1 (PD-L1, also called B7-H1 or CD274), which inhibits activation and effector functions of T cells. In the inhibitory mechanism, PD-1 and its ligands have become an important pathway for tumor cells to suppress activated T cells in the tumor microenvironment.

Programmed death receptor 1 (PD-1) plays an important role in immune regulation and maintenance of peripheral tolerance. PD-1 is expressed primarily in activated T and B cells and functions to inhibit the activation of lymphocytes, which is a normal peripheral tissue tolerance mechanism of the immune system that prevents over-reactive immunity. However, the activated T cells infiltrated in the tumor microenvironment highly express PD-1 molecules, and inflammatory factors secreted by the activated leukocytes can induce the tumor cells to highly express ligands PD-L1 and PD-L2 of PD-1, resulting in the continuous activation of the PD-1 pathway of the activated T cells in the tumor microenvironment, and the suppression of T cell function to kill tumor cells. Therapeutic PD-1 antibodies can block this pathway, partially restore the function of T cells, and enable the activated T cells to continuously kill tumor cells.

Blocking the PD-1/PD-L1 pathway has proven to be an effective way to induce a durable anti-tumor response in various cancer indications over the last decade. Monoclonal antibodies (mAbs) blocking the PD/PD-L1 pathway can enhance activation and effector functions of tumor specific T cells, reduce tumor burden, and improve survival rate. Between 2014 and 2017, the FDA has approved 2 anti-PD-1 monoclonal antibodies (nivolumab and pembrolizumab) and 3 anti-PD-L1 monoclonal antibodies (atezolizumab, avelumab and durvalumab) for treating human tumors. Melanoma was the first indication for which nivolumab and pembrolizumab were approved in 2014.

Neuroendocrine neoplasms (NENs) are neoplasms that originate from neuroendocrine cells. Neuroendocrine cells are a large group of cells in the body that have neuroendocrine phenotypes and can produce a variety of hormones. Neuroendocrine cells are distributed throughout the body, so neuroendocrine neoplasms can develop in any part of the body, and the most common ones are neuroendocrine neoplasms of the digestive system, such as the stomach, intestine and pancreas, which account for about 2/3 of all neuroendocrine neoplasms. The incidence of neuroendocrine neoplasms in European and American populations is 2.5-5/100,000, which has been increased by 5 times in the past 30 years. The incidence of neuroendocrine neoplasms is increasing more rapidly than that of other neoplasms. Moreover, NENs are a highly heterogeneous tumor population whose biological behavior varies significantly from relatively inert to highly invasive. Individuals with recurrent or metastatic neuroendocrine neoplasms have limited treatment options and poor prognosis. Platinum-based chemotherapy is the current option of standard first-line treatment for advanced poorly differentiated NECs. After failure of the platinum-based regimen, the median PFS is less than 3-4 months and the median OS is less than 6 months at the second treatment. Tyrosine kinase inhibitors (sunitinib) and mTOR inhibitors (everolimus) have been proven to prolong the progression-free survival of well-differentiated NETs with a low mitotic index (Ki-67 index < 10%, grade 1-2). However, the response rate for those drugs is very low. Alternative second-line regimens include temozolamide in combination with FOLFIRI and FOLFOX, which, however, has limited clinical efficacy. The medical need for more effective and less toxic treatments for advanced NETs, especially for NETs with a high mitotic index (Ki-67 index > 10%) is not yet met.

### SUMMARY

In one aspect, the present invention provides a method for treating an individual with a neuroendocrine neoplasm, which comprises administering to the individual a therapeutically effective amount of an anti-PD-1 antibody or an antigen-binding fragment thereof.

In a second aspect, the present invention provides use of an anti-PD-1 antibody or an antigen-binding fragment thereof in the preparation of a medicament for treating a patient with a neuroendocrine neoplasm.

In one or more embodiments, the neuroendocrine neoplasm described herein is recurrent or metastatic.

In one or more embodiments, the neuroendocrine neoplasm described herein is a neuroendocrine neoplasm with a proliferation index ki-67 ≥ 10%.

In one or more embodiments, the neuroendocrine neoplasm described herein includes a well-differentiated neuroendocrine tumor (NET) and/or a poorly differentiated neuroendocrine carcinoma (NEC).

In one or more preferred embodiments, the neuroendocrine neoplasm described herein is a poorly differentiated neuroendocrine carcinoma (NEC) with ki-67 ≥ 10%.

In one or more preferred embodiments, the neuroendocrine neoplasm described herein is a well-differentiated neuroendocrine tumor (NET) with ki-67 ≥ 10%.

In one or more embodiments, the individual with a neuroendocrine neoplasm described herein has positive PD-L1 expression (PD-L1 expression ≥ 1%) in a tumor tissue section by immunohistochemical staining analysis. By way of a preferred embodiment, the individual with a neuroendocrine neoplasm described herein has PD-L1 expression of ≥ 10% in a tumor tissue section by immunohistochemical staining analysis.

In one or more embodiments, the individual with a neuroendocrine neoplasm described herein has a high tumor mutation burden (TMB-H). By way of a preferred embodiment, the individual with a neuroendocrine neoplasm described herein has a tumor mutation burden (TMB) of ≥ 9.9 mutations/million base pairs.

In one or more embodiments, the individual with a neuroendocrine neoplasm described herein has positive PD-L1 expression in a tumor tissue section by immunohistochemical staining analysis and a high tumor mutation burden (TMB-H). In one or more embodiments, the individual with a neuroendocrine neoplasm described herein has PD-L1 expression of ≥ 10% in a tumor tissue section by immunohistochemical staining analysis and a tumor mutation burden (TMB) of ≥ 9.9 mutations/million base pairs.

In one or more embodiments, the individual with a neuroendocrine neoplasm described herein has microsatellite instability (MSI-H). In one or more embodiments, the individual with a neuroendocrine neoplasm described herein has microsatellite instability (MSI-H) and a high tumor mutation burden (TMB-H). In one or more embodiments, the individual with a neuroendocrine neoplasm described herein has microsatellite instability (MSI-H) and a tumor mutation burden (TMB) of ≥ 9.9 mutations/million base pairs.

In certain embodiments, the individual has received a previous therapy. In certain embodiments, the individual has received a standard therapy. In certain embodiments, the individual has no history of autoimmune diseases; in certain embodiments, the individual is not diagnosed with immunodeficiency; in certain embodiments, the individual has not received systemic steroid therapy for the past 4 weeks; in certain embodiments, the individual has not been treated with myelostimulating factor for the past 2 weeks; in certain embodiments, the individual has not received monoclonal antibody therapy/chemotherapy/targeted small molecule therapy for the past 4 weeks; in certain embodiments, the individual has not received radiotherapy for the past 4 weeks; in certain embodiments, the individual has no interstitial lung disease; in certain embodiments, the individual has no history of HIV infection; in certain embodiments, the individual has no known active hepatitis B or C virus infection/history of tuberculosis; in certain embodiments, the individual has no other known progressive malignancies/active brain metastases/cancerous meningitis; in certain embodiments, the individual has not received previous immune checkpoint blockade therapy.

In one or more embodiments, the anti-PD-1 antibody or the antigen-binding fragment thereof is administered at a single dose of about 0.1 mg/kg body weight to about 10.0 mg/kg body weight, e.g., about 0.1 mg/kg body weight, about 0.3 mg/kg body weight, about 1 mg/kg body weight, about 2 mg/kg body weight, about 3 mg/kg body weight, about 5 mg/kg body weight or 10 mg/kg body weight, or selected from a fixed dose of about 120 mg to about 480 mg, e.g., a fixed dose of about 120 mg, 240 mg, 360 mg or 480 mg.

In one or more embodiments, the anti-PD-1 antibody or the antigen-binding fragment thereof is administered at a frequency of about once every week, once every two weeks, once every three weeks, once every four weeks or once a month, preferably once every two weeks.

In one or more embodiments, the anti-PD-1 antibody or the antigen-binding fragment thereof is administered at a single dose of 1 mg/kg body weight, 3 mg/kg body weight or 10 mg/kg body weight, or of a fixed dose of 240 mg or 480 mg once every two weeks.

In one or more embodiments, the anti-PD-1 antibody or the antigen-binding fragment thereof is administered parentally, e.g., by intravenous infusion, in a liquid dosage form, e.g., an injection.

In one or more embodiments, the anti-PD-1 antibody or the antigen-binding fragment thereof can be administered in cycles of one week, two weeks, three weeks, one month, two months, three months, four months, five months, half a year or longer; optionally, the cycles each can be identical or different, and at identical or different intervals.

In one or more embodiments, in the treatment with the anti-PD-1 antibody, the anti-PD-1 antibody or the antigen-binding fragment thereof is administered at a therapeutically effective dose of 0.1 mg/kg to 10.0 mg/kg, or of a fixed dose of 240 mg, intravenously once every 2-6 weeks. In some embodiments, the anti-PD-1 antibody or the antigen-binding fragment thereof is administered at 1 mg/kg, 3 mg/kg or 10 mg/kg, or at a fixed dose of 240 mg once every 2-6 weeks.

In a third aspect, the present invention provides a kit for treating an individual with a neuroendocrine neoplasm, which comprises: (a) an antibody or an antigen-binding fragment thereof that specifically binds to human PD-1; and (b) instructions for using the anti-PD-1 antibody or the antigen-binding fragment thereof alone to treat the neuroendocrine neoplasm in the individual.

In a fourth aspect, the present invention provides a method for predicting the therapeutic effect of an anti-PD-1 antibody or an antigen-binding fragment thereof on a neuroendocrine neoplasm, which comprises detecting a biomarker in peripheral blood or tumor tissue of an individual prior to treatment, wherein the biomarker include, but is not limited to, an *ARID1A* mutation. In one or more embodiments, the presence of the *ARID1A* gene mutation indicates that the patient with the neoplasm is suitable for treatment with an anti-PD-1 antibody.

In a fifth aspect, the present invention provides a kit for predicting the therapeutic effect of an anti-PD-1 antibody or an antigen-binding fragment thereof on a neuroendocrine neoplasm, which comprises (a) an agent for detecting an *ARID1A* gene mutation in peripheral blood or tumor tissue of an individual; (b) instructions for using the agent for detecting the *ARID1A* gene mutation in peripheral blood or tumor tissue to predict the therapeutic effect of an anti-PD-1 antibody or an antigen-binding fragment thereof.

In the use, method and kit described herein, the neuroendocrine neoplasm is recurrent or metastatic. By way of a preferred embodiment, the neuroendocrine neoplasm is a neuroendocrine neoplasm with a proliferation index ki-67 ≥ 10%. By way of a preferred embodiment, the neuroendocrine neoplasm is a well-differentiated neuroendocrine tumor or a poorly differentiated neuroendocrine carcinoma. By way of a preferred embodiment, the neuroendocrine neoplasm is a poorly differentiated neuroendocrine carcinoma (NEC) with ki-67 ≥ 10%. By way of a preferred embodiment, the neuroendocrine neoplasm is a well-differentiated neuroendocrine tumor (NET) with ki-67 ≥ 10%. By way of a preferred embodiment, the neuroendocrine neoplasm has positive PD-L1 expression (≥ 1%) in a tumor tissue section by immunohistochemical staining analysis. By way of a preferred embodiment, the neuroendocrine neoplasm has PD-L1 expression of ≥ 10% in a tumor tissue section by immunohistochemical staining analysis. By way of a preferred embodiment, the neuroendocrine neoplasm has a high tumor mutation burden (TMB-H). By way of a preferred embodiment, the neuroendocrine neoplasm has a tumor mutational burden (TMB) of ≥ 9.9 mutations/million base pairs. By way of a preferred embodiment, the neuroendocrine neoplasm has microsatellite instability (MSI-H). By way of a preferred embodiment, the neuroendocrine neoplasm has positive PD-L1 expression in a tumor tissue section by immunohistochemical staining analysis and a high tumor mutation burden (TMB-H). By way of a preferred embodiment, the neuroendocrine neoplasm has positive PD-L1 expression of ≥ 10% in a tumor tissue section by immunohistochemical staining analysis and a tumor mutation burden (TMB) of ≥ 9.9 mutations/million base pairs. By way of a preferred embodiment, the individual with a neuroendocrine neoplasm described herein has microsatellite instability (MSI-H) and a tumor mutation burden (TMB) of ≥ 9.9 mutations/million base pairs. By way of a preferred embodiment, the neuroendocrine neoplasm has microsatellite instability (MSI-H) and positive PD-L1 expression in a tumor tissue section by immunohistochemical staining analysis. By way of a preferred embodiment, the neuroendocrine neoplasm has microsatellite instability (MSI-H) and positive PD-L1 expression of ≥ 10% in a tumor tissue section by immunohistochemical staining analysis.

In the use, method and kit described herein, the anti-PD-1 antibody is a monoclonal antibody or an antigen-binding fragment thereof. In certain embodiments, the anti-PD-1 antibody specifically binds to PD-1 and blocks the binding of PD-L1 and/or PD-L2 to PD-1. In certain embodiments, the anti-PD-1 antibody specifically binds to PD-L1 or/and PD-L2 and blocks the binding of PD-L1 and/or PD-L2 to PD-1.

In one or more embodiments, the anti-PD-1 antibody or the antigen-binding fragment thereof comprises at least one complementarity determining region (CDR), wherein the CDR comprises an amino acid sequence selected from SEQ ID NOs: 1. 2, 3, 4, 5 and 6.

In one or more embodiments, the anti-PD-1 antibody or the antigen-binding fragment thereof comprises light chain complementarity determining regions (LCDRs), wherein the LCDRs comprise amino acid sequences set forth in SEQ ID NOs: 1,2 and 3.

In one or more embodiments, the anti-PD-1 antibody or the antigen-binding fragment thereof comprises heavy chain complementarity determining regions (HCDRs), wherein the HCDRs comprise amino acid sequences set forth in SEQ ID NO: 4, 5 and 6.

In one or more embodiments, the anti-PD-1 antibody or the antigen-binding fragment thereof comprises light chain complementarity determining regions (LCDRs) and heavy chain complementarity determining regions (HCDRs), wherein the LCDRs comprise amino acid sequences set forth in SEQ ID NOs: 1,2 and 3, and the HCDRs comprise amino acid sequences set forth in SEQ ID NOs: 4, 5 and 6.

In one or more embodiments, the anti-PD-1 antibody comprises a light chain variable region (VL) and/or a heavy chain variable region (VH), wherein the VL comprises an amino acid sequence set forth in SEQ ID NO: 7, and the VH comprises an amino acid sequence set forth in SEQ ID NO: 8.

In one or more embodiments, the anti-PD-1 antibody is an anti-PD-1 antibody comprising a light chain and a heavy chain, wherein the light chain comprises an amino acid sequence set forth in SEQ ID NO: 9, and the heavy chain comprises an amino acid sequence set forth in SEQ ID NO: 10.

In one or more embodiments, the anti-PD-1 antibody is selected from nivolumab, pembrolizumab, toripalima, sintilimab, camrelizumab, tislelizumab, cemiplimab, and a combination thereof.

In a sixth aspect, the present invention provides a method for predicting the therapeutic effect of an anti-PD-1 antibody on an individual with a neoplasm, which comprises detecting a biomarker in tumor tissue of the individual prior to treatment, wherein the biomarker is a chromosomal gene rearrangement.

In a seventh aspect, the present invention provides a kit for predicting the therapeutic effect of an anti-PD-1 antibody or an antigen-binding fragment thereof on a neoplasm of an individual, which comprises (a) an agent for detecting a chromosomal gene rearrangement in tumor tissue of an individual; (b) instructions for using the agent for detecting the chromosomal gene rearrangement in the tumor tissue of the individual to predict the therapeutic effect of an anti-PD-1 antibody or an antigen-binding fragment thereof.

In the method and kit described herein, the individual is a human; and the neoplasm is a solid tumor. By way of one or more preferred embodiments, the individual with a neoplasm is a patient with a neuroendocrine neoplasm. In one or more particular embodiments, the individual with a neoplasm is a patient with a neuroendocrine neoplasm with a proliferation index ki-67 ≥ 10%. In one or more particular embodiments, the patient with a neoplasm is a patient with a poorly differentiated neuroendocrine carcinoma (NEC) with ki-67 ≥ 10%. In one or more particular embodiments, the patient with a neoplasm is a patient with a well-differentiated neuroendocrine tumor (NET) with ki-67 ≥ 10%.

In another one or more embodiments of the use, method and kit described herein, the subject is a patient who has a neuroendocrine neoplasm and has not received a previous immunotherapy.

In any one of the embodiments of the use, method and kit described above, the anti-PD-1 antibody is toripalimab.

### BRIEF DESCRIPTION OF THE DRAWINGS

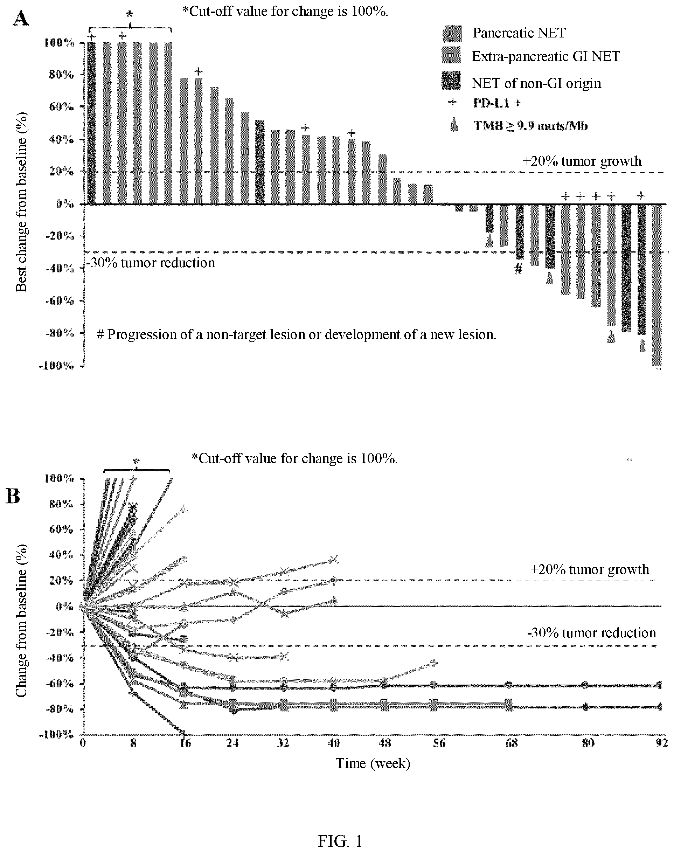

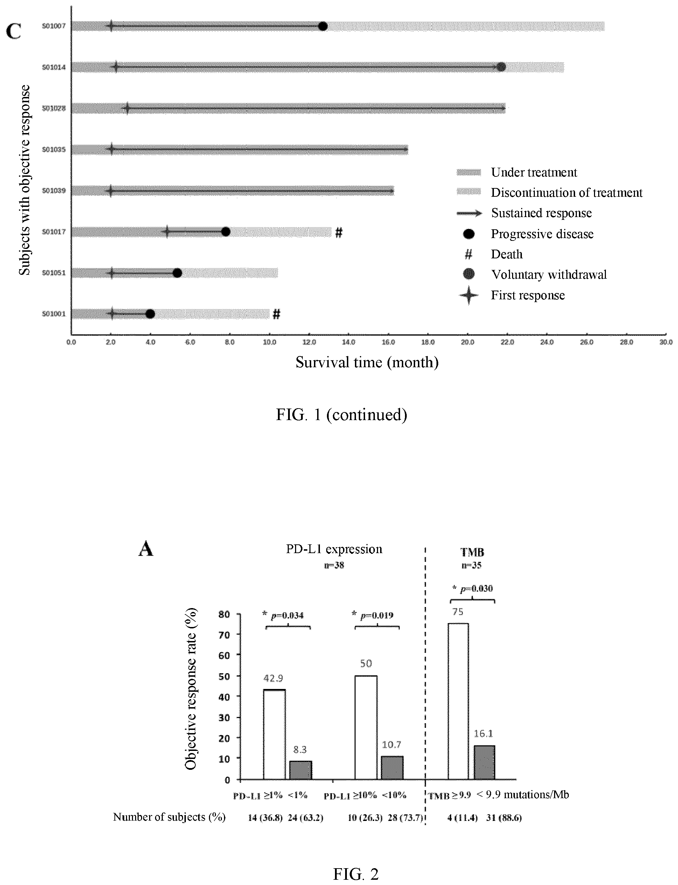

FIG. 1: results of the evaluation of clinical responses according to RECIST v1.1. A: maximum changes in tumor size from baseline evaluated for patients with at least one posttreatment imaging evaluation (n = 38), wherein the length of the bar represents the maximum decrease or minimum increase of the target lesion; B: change in tumor burden in the individuals from baseline over time; C: the response event and duration of response of patients who have objective response.

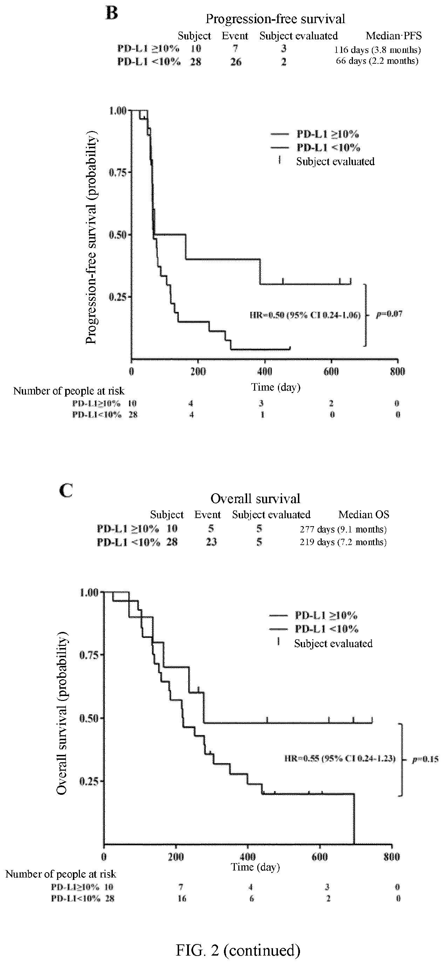

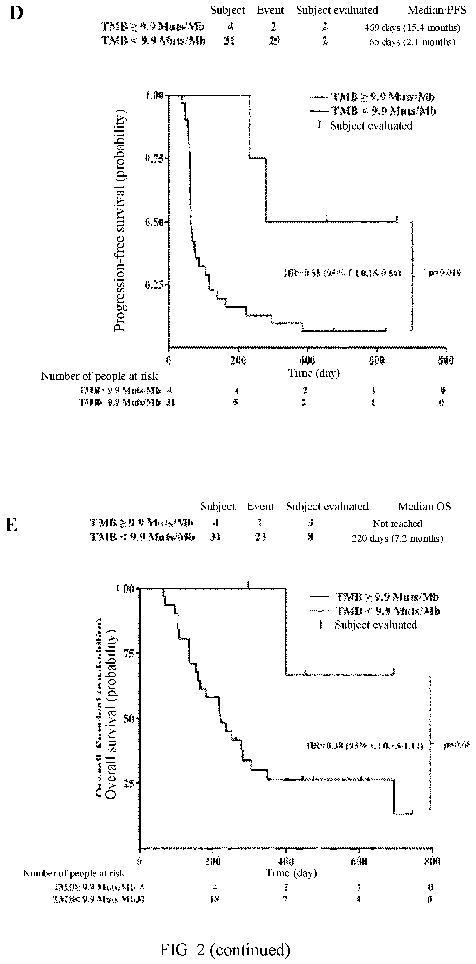

FIG. 2: relationship between the tumor PD-L1 expression and tumor mutation burden (TMB) and clinical response. A: PD-L1 positive refers to the presence of any tumor cells or stromal cells with membrane staining intensity ≥ 1% by SP142 staining; TMB is calculated by whole exome sequencing of total somatic mutations within the coding region, with the top 10% of TMB values of 9.9 mutations/million base pairs as a cut-off value; B: progression-free survival/RSCIST v1.1 of subjects with PD-L1 ≥ 10% vs. PD-L1 < 10%; C: overall survival of subjects with PD-L1 ≥ 10% vs. PD-L1 < 10%; D: progression-free survival/RSCIST v1.1 of subjects with TMB ≥ 9.9 mutations/million base pairs vs. TMB < 9.9 mutations/million base pairs; E: overall survival of subjects with TMB ≥ 9.9 mutations/million base pairs vs. TMB < 9.9 mutations/million base pairs.

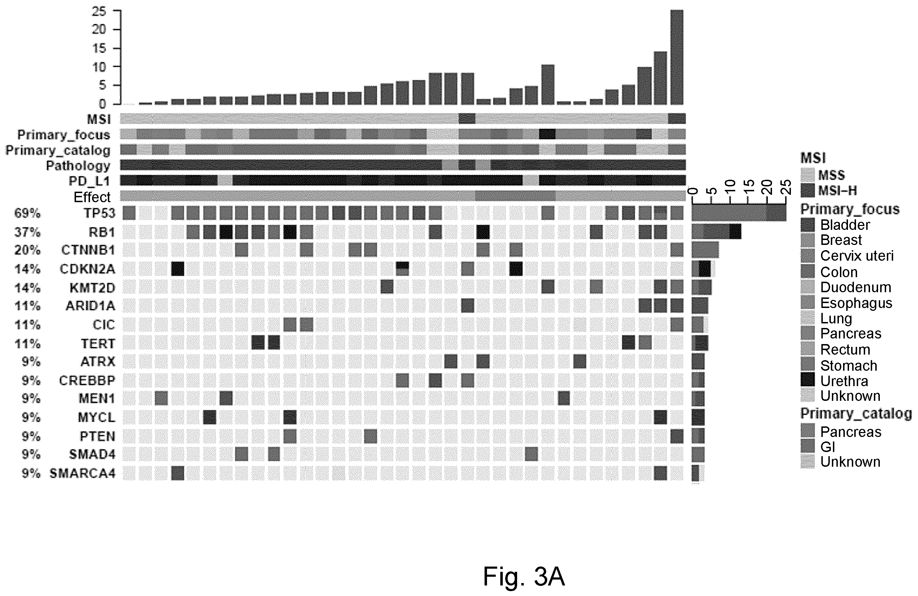

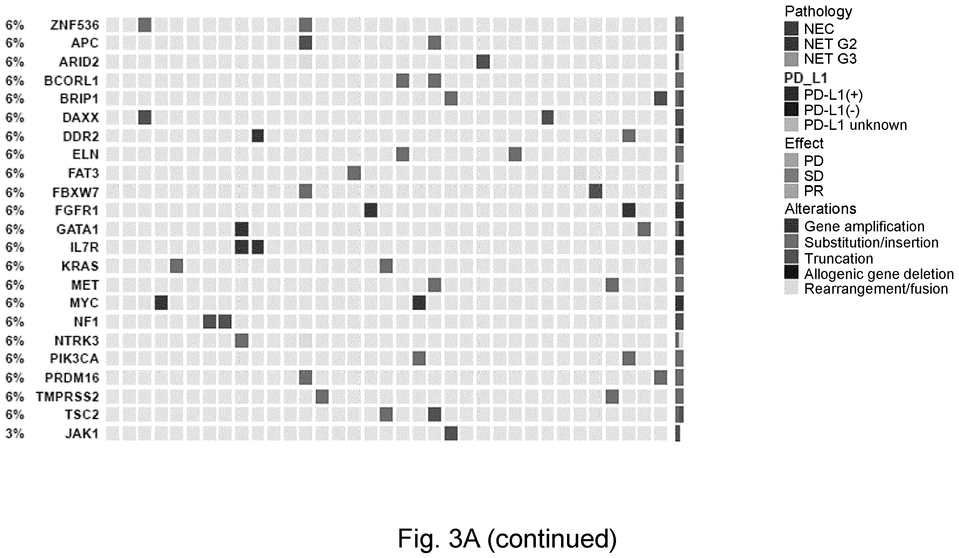

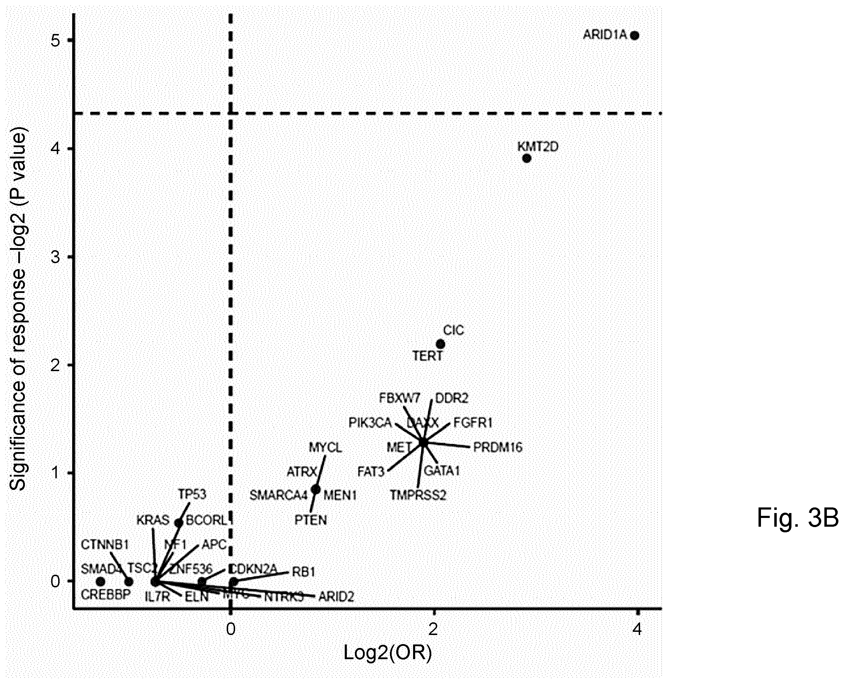

FIG. 3: gene variations of 35 subjects identified by WES. A: subjects were grouped by PD-L1 expression, primary tumor tissue and clinical response; B: correlation of gene variation with clinical objective response.

### DETAILED DESCRIPTION

The present invention relates to a method for treating a neuroendocrine neoplasm. The method of the present invention comprises administering to an individual in need a therapeutically effective amount of an anti-PD-1 antibody or an antigen-binding fragment thereof. The present invention also relates to a method for predicting the therapeutic effect of an anti-PD-1 antibody on an individual with cancer, particularly a neuroendocrine neoplasm, using a biomarker.

### Terminology

In order to facilitate the understanding of the present invention, some technical and scientific terms are specifically defined below. Unless otherwise specifically defined herein, all technical and scientific terms used herein have the same meaning as commonly understood by those of ordinary skill in the art to which the present invention belongs.

"Administering", "giving" and "treating" refers to introducing a composition comprising a therapeutic agent into a subject using any one of a variety of methods or delivery systems known to those skilled in the art. Routes of administration of the anti-PD-1 antibody include intravenous, intramuscular, subcutaneous, intraperitoneal, spinal or other parenteral routes of administration, such as injection or infusion. "Parenteral administration" refers to modes of administration apart from enteral or local administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion, and *in vivo* electroporation.

An "adverse event" (AE) described herein is any adverse and often unintended or undesirable sign, symptom, or disease associated with the use of medical treatment. For example, an adverse event may be associated with the activation of the immune system or the amplification of immune system cells in response to treatment. The medical treatment may have one or more related AEs, and the AEs each may have identical or different severity levels.

"Tumor burden" refers to the total amount of tumor mass distributed throughout the body. Tumor burden refers to the total number of cancer cells or the total size of the tumor throughout the body. Tumor burden can be determined by a variety of methods known in the art, such as measuring the size of a tumor using calipers after the tumor is removed from a subject, or using imaging techniques (e.g., ultrasound, bone scanning, computed tomography (CT), or magnetic resonance imaging (MRI) scanning) when the tumor is *in vivo.*

The term "tumor size" refers to the total size of a tumor, which can be measured as the length and width of the tumor. Tumor size can be determined by a variety of methods known in the art, such as measuring the size of a tumor using calipers after the tumor is removed from a subject, or using imaging techniques (e.g., bone scanning, ultrasound, CT, or MRI scanning) when the tumor is *in vivo.*

The terms "subject" and "individual" include any organism, preferably an animal, more preferably a mammal (such as rat, mouse, dog, cat and rabbit), and most preferably a human. The terms "subject" and "individual" are used interchangeably herein.

An "antibody" described herein refers to any form of antibody that achieves a desired biological or binding activity. Therefore, it is used in the broadest sense, but is not limited to, monoclonal antibodies, polyclonal antibodies, multispecific antibodies, humanized full-length human antibodies, chimeric antibodies and camelid single-domain antibodies. The "antibody" specifically binds to an antigen and comprises at least two heavy (H) chains and two light (L) chains interconnected by disulfide bonds. Each heavy chain comprises a heavy chain variable region (VH) and a heavy chain constant region comprising three constant domains CH1, CH2 and CH3. Each light chain comprises a light chain variable region (VL) and a light chain constant region comprising one constant domain CL. The VH and VL regions can be further divided into hypervariable regions termed complementarity determining regions (CDRs), which are scattered over more conserved regions termed framework regions (FRs). Generally, both light and heavy chain variable domains comprise FR1, CDR1, FR2, CDR2, FR3, CDR3 and FR4 from N-terminus to C-terminus. Amino acids are typically assigned to each domain according to the following definitions: Sequences of Proteins of Immunological Interest, Kabat et al.; National Institutes of Health, Bethesda, Md.; 5th edition; NIH publication No. 91-3242 (1991): Kabat (1978) Adv. Prot. Chem. 32:1-75; Kabat et al., (1977) J. Biol. Chem. 252:6609-6616; Chothia et al., (1987) J Mol. Biol. 196:901-917 or Chothia et al., (1989) Nature 341:878-883.

The carboxyl-terminal portion of the heavy chain can define a constant region primarily responsible for effector function. Human light chains are generally classified as κ and λ chains. Human heavy chains are generally classified as µ, δ, γ, α or ε chains, and isotypes of the antibody are defined as IgM, IgD, IgG, IgA and IgE, respectively. IgG subclass is well known to those skilled in the art and includes, but is not limited to, IgG1, IgG2, IgG3 and IgG4.

The term "antibody" includes: naturally occurring and non-naturally occurring Abs; monoclonal and polyclonal Abs; chimeric and humanized Abs; human or non-human Abs; fully synthetic Abs; and single chain Abs. Non-human Abs can be humanized by recombinant methods to reduce their immunogenicity in humans.

Unless otherwise specifically indicated, an "antibody fragment" or "antigen-binding fragment" described herein refers to an antigen-binding fragment of an antibody, i.e., an antibody fragment that retains the ability of a full-length antibody to specifically bind to an antigen, e.g., a fragment that retains one or more CDR regions. Examples of an antigen-binding fragment include, but are not limited to, Fab, Fab', F(ab')₂, and Fv fragments; a diabody; a linear antibody; a single-chain antibody molecule; and a nanoantibody and a multispecific antibody formed from fragments of the antibody.

A "chimeric antibody" refers to an antibody and a fragment thereof in which a portion of the heavy and/or light chain is identical with or homologous to corresponding sequences of an antibody derived from a particular species (e.g., human) or belonging to a particular antibody class or subclass, while the remainder of the chain is identical with or homologous to corresponding sequences of an antibody derived from another species (e.g., mouse) or belonging to another antibody class or subclass, so long as they exhibit the desired biological activity.

A "human antibody" refers to an antibody that comprises only human immunoglobulin sequences. A human antibody may contain a murine carbohydrate chain if it is produced in mice, mouse cells, or hybridomas derived from mouse cells. Similarly, "mouse antibody" or "rat antibody" refers to an antibody that comprises only mouse or rat immunoglobulin sequences, respectively.

A "humanized antibody" refers to an antibody form containing sequences from both non-human (e.g., murine) and human antibodies. Such antibodies contain minimal sequences derived from non-human immunoglobulins. Typically, a humanized antibody will comprise substantially all of at least one and typically two variable domains, in which all or substantially all of the hypervariable loops correspond to those of a non-human immunoglobulin and all or substantially all of the FR regions are those of a human immunoglobulin. The humanized antibody optionally also comprises at least one portion of an immunoglobulin constant region (Fc), typically a human immunoglobulin constant region.

The term "immunotherapy" refers to the treatment of a subject with a disease or at risk of infection or disease recurrence by a method that includes inducing, enhancing, suppressing or otherwise modifying an immune response. The "treatment" or "therapy" of a subject refers to any type of intervention or process performed on the subject, or the administration of an active agent to the subject, with the purpose of reversing, alleviating, ameliorating, slowing or preventing the onset, progression, severity, or recurrence of symptoms, complications or conditions, or biochemical indicators associated with the disease.

A "programmed death receptor-1 (PD-1)" refers to an immunosuppressive receptor belonging to the CD28 family. PD-1 is expressed primarily on previously activated T cells *in vivo* and binds to two ligands, PD-L1 and PD-L2. The term "PD-1" used herein includes human PD-1 (hPD-1), variants, isotypes, and species homologs of hPD-1, and analogs having at least one common epitope with hPD-1.

A "therapeutically effective amount" or "therapeutically effective dose" of a medicament or therapeutic agent is any amount of the medicament that, when used alone or in combination with an additional therapeutic agent, protects a subject from the onset of a disease or promotes the regression of a disease as evidenced by a decrease in the severity of disease symptoms, an increase in the frequency and duration of disease symptom-free phase, or the prevention of injury or disability resulting from the affliction of the disease. The ability of a therapeutic agent to promote the regression of a disease can be assessed using a variety of methods known to those skilled in the art, such as in human subjects during clinical trials, in animal model systems that predict human efficacy, or by determining the activity of the agent in an *in vitro* assay.

A therapeutically effective amount of a medicament includes a "prophylactically effective amount" which is any amount of a medicament that, when administered alone or in combination with an anti-neoplastic agent, inhibits the development or recurrence of cancer to a subject at risk of developing cancer or a subject having cancer recurrence.

A "biotherapeutic agent" refers to a biomolecule, such as an antibody or fusion protein, that blocks ligand/receptor signaling in any biological pathway that supports tumor maintenance and/or growth or inhibits an anti-tumor immune response.

Unless otherwise specifically indicated, "CDR" used herein refers to a complementarity determining region of the immunoglobulin variable region defined using the Kabat numbering system.

A "therapeutic anti-PD-1 monoclonal antibody" refers to an antibody that specifically binds to the mature form of a specific PD-1 expressed on the surface of certain mammalian cells. Mature PD-1 does not have a secretory leader sequence, or leader peptide. The terms "PD-1" and "mature PD-1" are used interchangeably herein and are to be understood as the same molecule unless otherwise specifically defined, or clearly seen from the context.

A therapeutic anti-human PD-1 antibody or anti-hPD-1 antibody described herein refers to a monoclonal antibody that specifically binds to mature human PD-1.

A "framework region" or "FR" described herein refers to the immunoglobulin variable region excluding CDR regions.

An "isolated antibody or antigen-binding fragment thereot" refers to a molecule that is in a purified state, and in this case, is substantially free of other biomolecules, such as nucleic acids, proteins, lipids, carbohydrates, or other materials (such as cell debris or growth medium).

An "individual", "patient" or "subject" refers to any single subject in need of a medical procedure or participating in a clinical trial, epidemiological study, or serving as a control, and is generally a mammal, including a human and other non-human mammals, such as horses, cows, dogs or cats.

The "RECIST 1.1 efficacy criteria" described herein refers to the definition of target injury and non-target injury described in Eisenhauver et al., E.A. et al., Eur. J Cancer 45:228-247(2009) in the context of the measured background.

The term "ECOG" score standard is an indicator of general health status and tolerance to treatment of patients from their physical strength. ECOG score standard for the physical strength is 0 points, 1 point, 2 points, 3 points, 4 points and 5 points. A score of 0 means that the motility is completely normal and has no difference from the motility before onset of disease. A score of 1 means that the person is free to walk and can engage in light physical activities, including general housework or office work, but not in heavy physical activities.

A "sustained response" refers to a sustained therapeutic effect following cessation of treatment with a therapeutic agent or combination therapy described herein. In some embodiments, the sustained response has a duration that is at least the same as the duration of treatment or at least 1.5, 2.0, 2.5 or 3 times the duration of the treatment.

A "tissue section" refers to a single portion or piece of a tissue sample, such as a tissue slice cut from a sample of normal tissue or a tumor.

"Treating" cancer described herein refers to administering a treatment regimen described herein (e.g., administration of an anti-PD-1 antibody or a combination therapy of an anti-PD-1 antibody with a CDK4/6 inhibitor) to a subject with or diagnosed with cancer to achieve at least one positive therapeutic effect (e.g., a decrease in cancer cell number, a decrease in tumor volume, a reduction in the rate of cancer cell infiltration into peripheral organs, or a reduction in the rate of tumor metastasis or tumor growth). Positive therapeutic effects in cancer can be measured in a variety of ways (see W. A. Weber, J. Nucl. Med., 50:1S-10S (2009)). For example, T/C ≤ 42% for tumor growth inhibition is the minimum level of anti-tumor activity according to the NCI criteria. It is considered that T/C (%) = median treated tumor volume/median control tumor volume × 100. In some embodiments, the therapeutic effect achieved by the combination of the present invention is any one of PR, CR, OR, PFS, DFS and OS. PFS (also called "time to tumor progression") refers to the length of time during and after treatment for which cancer does not grow, and includes the amount of time a patient experiences CR or PR and the amount of time a patient experiences SD. DFS refers to the length of time during and after treatment for which a patient remains disease-free. OS refers to an extension of life expectancy compared to an initial or untreated individual or a patient. In some embodiments, the response to the combination of the present invention is any one of PR, CR, PFS, DFS, OR or OS, assessed using RECIST 1.1 efficacy criteria. The treatment regimen of the combination of the present invention effective in treating a patient with cancer may vary depending upon a variety of factors such as the disease state, age, weight of the patient and the ability of the therapy to elicit an anti-cancer response in the subject. Embodiments of the present invention may not achieve an effective positive therapeutic effect in each subject, but should be effective and achieve a positive therapeutic effect in a statistically significant number of subjects.

The terms "mode of administration" and "dosing regimen" are used interchangeably and refer to the dosage and time of use of each therapeutic agent in the combination of the present invention.

The term "immunohistochemistry (IHC)" refers to a method for determining antigens (polypeptides and proteins) in tissue cells by developing chromogenic agents (fluoresceins, enzymes, metal ions, isotopes) that label antibodies through chemical reaction based on the principle that antigens specifically bind to antibodies, and performing localized, qualitative and relatively quantitative studies on those antigens. In some embodiments of the present invention, a tumor tissue sample from a subject is tested for PD-L1 prior to treatment with an anti-PD-1 antibody by staining the anti-human PD-L1 antibody SP142 from Roche (Cat No: M4422). In some embodiments, the presence of tumor cells with membrane staining ≥ 1% is defined as positive PD-L1 expression. In some embodiments, the membrane staining intensity of tumor cells is PD-L1 expression of ≥ 10%.

In the following paragraphs, various aspects of the present invention are further described in detail.

### Anti-PD-1 Antibody

A "PD-1 antibody" used herein refers to any chemical compound or biomolecule that binds to the PD-1 receptor, blocks the binding of PD-L1 expressed on cancer cells to PD-1 expressed on immune cells (T, B and NK cells), and preferably blocks the binding of PD-L2 expressed on cancer cells to PD-1 expressed on immune cells. Alternative nouns or synonyms for PD-1 and its ligands include: PDCD1, PD1, CD279 and SLEB2 for PD-1; PDCD1L1, PDL1, B7-H1, B7H1, B7-4, CD274 and B7-H for PD-L1; and PDCD1L2, PDL2, B7-DC and CD273 for PD-L2. In any of the therapy, medicament and use described herein for treating a human individual, the anti-PD-1 antibody blocks the binding of human PD-L1 to human PD-1, and preferably blocks the binding of both human PD-L1 and PD-L2 to human PD-1. The amino acid sequence of human PD-1 can be found at NCBI locus number: NP-_005009. The amino acid sequences of human PD-L1 and PD-L2 can be found at NCBI locus numbers: NP 054862 and NP 079515.

As used herein, unless otherwise indicated or described, when referring to the term "anti-PD-1 antibody", it includes antigen-binding fragments of the antibody.

The anti-PD-1 antibody suitable for any of the use, therapy, medicament and kit described herein has an immunosuppressive effect achieved by binding to PD-1 with high specificity and high affinity, blocking the binding of PD-L1/2 to PD-1 and inhibiting PD-1 signal transduction. In any of the use, therapy, medicament and kit disclosed herein, the anti-PD-1 antibody includes the full-length antibody itself, as well as an antigen-binding portion or fragment that binds to the PD-1 receptor and exhibits functional properties similar to an intact Ab in inhibiting ligand binding and upregulating the immune system. In some embodiments, the anti-PD-1 antibody or the antigen-binding fragment thereof is an anti-PD-1 antibody or an antigen-binding fragment thereof that cross-competes for binding to human PD-1 with toripalimab. In other embodiments, the anti-PD-1 antibody or the antigen-binding fragment thereof is a chimeric, humanized or human Ab or an antigen-binding fragment thereof. In certain embodiments for treating a human individual, the Ab is a humanized Ab.

In some embodiments, the anti-PD-1 antibody that can be used in any of the use, therapy, medicament and kit described herein includes a monoclonal antibody (mAb) or an antigen-binding fragment thereof that specifically binds to PD-1, and preferably specifically binds to human PD-1. The mAb may be a human antibody, a humanized antibody, or a chimeric antibody, and may include a human constant region. In some embodiments, the constant region is selected from human IgG1, IgG2, IgG3 and IgG4 constant regions; preferably, the anti-PD-1 antibody or the antigen-binding fragment thereof suitable for use in any of the use, therapy, medicament and kit described herein comprises a heavy chain constant region of human IgG1 or IgG4 isotype, more preferably a human IgG4 constant region. In some embodiments, the sequence of the IgG4 heavy chain constant region of the anti-PD-1 antibody or the antigen-binding fragment thereof comprises the S228P mutation that replaces a serine residue in the hinge region with a proline residue that is typically present at the corresponding position of an antibody of IgG1 isotype.

Preferably, in any one of the embodiments of the use, method and kit described herein, the anti-PD-1 antibody is a monoclonal antibody or an antigen-binding fragment thereof comprising at least one complementarity determining region (CDR), wherein the CDR comprises an amino acid sequence selected from SEQ ID NOs: 1. 2, 3, 4, 5 and 6.

More preferably, in any one of the embodiments of the use, method and kit described herein, the anti-PD-1 antibody is a monoclonal antibody or an antigen-binding fragment thereof comprising light chain CDRs and/or heavy chain CDRs, wherein the light chain CDRs comprise amino acid sequences set forth in SEQ ID NOs: 1,2 and 3, and the heavy chain CDRs comprise amino acid sequences set forth in SEQ ID NOs: 4, 5 and 6.

Further preferably, in any one of the embodiments of the use, method and kit described herein, the anti-PD-1 antibody is a monoclonal antibody that specifically binds to human PD-1 and comprises: (a) a light chain variable region comprising an amino acid sequence set forth in SEQ ID NO: 7, and/or (b) a heavy chain variable region comprising an amino acid sequence set forth in SEQ ID NO: 8.

Further preferably, in any one of the embodiments of the use, method and kit described herein, the anti-PD-1 antibody specifically binds to human PD-1 and comprises: (a) a light chain comprising an amino acid sequence set forth in SEQ ID NO: 9, and/or (b) a heavy chain comprising an amino acid sequence set forth in SEQ ID NO: 10.

Table A below provides the amino acid sequence numbering of the light chain CDRs and heavy chain CDRs of an exemplary anti-PD-1 antibody mAb for use in the use, method and kit described herein:

**Table A: Light and heavy chain CDRs (Kabat) of an exemplary anti-human PD-1 antibody**

| | |
|---|---|
| LCDR1 | SEQ ID NO: 1 |
| LCDR2 | SEQ ID NO: 2 |
| LCDR3 | SEQ ID NO: 3 |
| HCDR1 | SEQ ID NO: 4 |
| HCDR2 | SEQ ID NO: 5 |
| HCDR3 | SEQ ID NO: 6 |

An example of anti-PD-1 antibodies that bind to human PD-1 and can be used in the use, therapy, medicament and kit described herein is described in WO2014206107. Human PD-1 mAbs that can be used as anti-PD-1 antibodies in the use, therapy, medicament and kit described herein include any one of the anti-PD-1 antibodies described in WO2014206107, including toripalimab (a humanized IgG4 mAb having the structure described in WHO Drug Information; 32(2):372-373 (2018) and comprising light and heavy chain amino acid sequences set forth in SEQ ID NOs: 9 and 10). In a preferred embodiment, the anti-PD-1 antibody that can be used in any one of the use, therapy, medicament and kit described herein is selected from humanized antibodies 38, 39, 41 and 48 described in WO2014206107. In a particularly preferred embodiment, the anti-PD-1 antibody that can be used in any one of the use, therapy, medicament and kit described herein is toripalimab.

In certain embodiments, the anti-PD-1 antibody that can be used in the use, method and kit described herein also includes nivolumab, pembrolizumab, toripalimab, sintilimab, camrelizumab, tislelizumab, cemiplimab or a combination thereof.

In certain embodiments, the anti-PD-1 antibody that can be used in the use, method and kit described herein also includes anti-PD-Ll monoclonal antibodies that specifically bind to PD-L1 to block the binding of PD-L1 to PD-1, such as atezolizumab, avelumab and durvalumab.

"PD-L1" expression or "PD-L2" expression described herein refers to any detectable expression level of a specific PD-L protein on the surface of a cell or a specific PD-L mRNA within a cell or tissue. PD-L protein expression can be detected in IHC analysis of tumor tissue sections or by flow cytometry using diagnostic PD-L antibodies. Alternatively, PD-L protein expression of tumor cells can be detected by PET imaging using a binding agent that specifically binds to a desired PD-L target (such as PD-L1 or PD-L2).

Methods for quantifying PD-L1 protein expression in IHC analysis of tumor tissue sections are found in, but are not limited to, Thompson, R. H. et al., PNAS 101(49):17174-17179 (2004); Taube, J. M. et al., Sci TranslMed 4, 127ra37 (2012); and Topalian, S. L. et al., New Eng. J. Med. 366(26): 2443-2454 (2012), and the like.

In one method, a simple binary endpoint of positive or negative PD-L1 expression is adopted, where the positive expression is defined by the percentage of tumor cells showing histological evidence of cell surface membrane staining. The case where tumor cells on a tumor tissue section account for at least 1% of the total tumor cells is defined as positive PD-L1 expression.

In another method, PD-L1 expression in the tumor tissue section is quantified in tumor cells as well as in infiltrating immune cells. The percentage of tumor cells and infiltrating immune cells exhibiting membrane staining are quantified individually as < 1%, 1% to 10%, and subsequent 10% to 100%. For tumor cells, the PD-L1 expression is counted as negative if the score is < 1%, counted as positive if the score is ≥ 1%, and counted as high expression if the score is ≥ 10%.

In some embodiments, the expression level of PD-L1 by malignant cells and/or by infiltrating immune cells within the tumor is determined to be "overexpressed" or "elevated" based on comparison with the expression level of PD-L1 by an appropriate control. For example, the protein or mRNA expression level of control PD-L1 can be a quantified level in nonmalignant cells of the same type or in sections from matched normal tissue.

### Disease and Treatment Thereof

The present invention relates to the treatment of a neuroendocrine neoplasm. The present invention comprises administering to a patient with a neuroendocrine neoplasm in need an anti-PD-1 antibody or an antigen-binding fragment thereof alone.

In the present invention, the term "neuroendocrine neoplasm (NEN)" refers to neoplasms that originate from neuroendocrine cells at any part of the body, which are widely distributed in the human body and mostly occurs in the stomach, intestine and pancreas. According to the parts at which the neoplasms occur, the neuroendocrine neoplasms can be classified as: 1) pancreatic neuroendocrine neoplasms; 2) gastrointestinal neuroendocrine neoplasms; and 3) neuroendocrine neoplasms in other parts. In 2017, WHO classified NENs into two major types, i.e., well-differentiated neuroendocrine tumors (NETs) and poorly differentiated neuroendocrine carcinomas (NECs). In many organ systems, NETs are classified as G1, G2 and G3, corresponding to low, medium and high grade, respectively, based on mitotic counts, Ki67 marker counts and the presence or absence of necrosis. In some organs, certain names have actually reflected a grade (e.g., in lung and thymus, G1 for carcinoid, and G2 for atypical carcinoid). In the present invention, the neuroendocrine neoplasm may be a recurrent or metastatic neuroendocrine neoplasm, preferably a poorly differentiated neuroendocrine carcinoma (NEC) or a moderately and well-differentiated neuroendocrine tumor (NET).

Preferably, the method, use and pharmaceutical composition according to any one of the embodiments of the present invention are especially suitable for neuroendocrine neoplasms with a proliferation index Ki-67 ≥ 10%, and/or neuroendocrine neoplasms with positive PD-L1 expression as shown in tumor tissue section analysis results, and/or neuroendocrine neoplasms with a high tumor mutation burden (TMB-H).

The term "Ki-67" used herein refers to a proliferating cell-related nuclear antigen that is used primarily in the examination of a neoplasm to determine the degree of malignancy of the neoplasm. A higher value of Ki-67 indicates more active cell proliferation, faster tumor growth, poorer tissue differentiation, and more sensitivity to chemotherapy. In the field of neuroendocrine neoplasms, a Ki-67 index is used for analyzing the degree of malignancy of the tumor. Generally, if the Ki-67 index is between 0% and 2%, the degree of malignancy is called G1; if the index is between 2% and 20%, the degree of malignancy is called G2; and if the index is greater than 20%, the degree of malignancy is called G3. G1, G2 and G3 indicate the concepts of good, average, and poor, respectively.

Herein, preferably, the neuroendocrine neoplasm with positive PD-L1 expression as shown in tumor tissue section analysis results refers to a neuroendocrine neoplasm with PD-L1 expression ≥ 1% as shown in tissue section analysis results, and more preferably, a neuroendocrine neoplasm with PD-L1 expression ≥ 10% as shown in tumor tissue section analysis results.

Herein, preferably, the neuroendocrine neoplasm is a neuroendocrine neoplasm with a high tumor mutation burden (TMB-H), and more preferably, a neuroendocrine neoplasm with a tumor mutation burden (TMB) ≥ 9.9 mutations/million base pairs.

Preferably, the neuroendocrine neoplasm suitable for use in the method, use and pharmaceutical composition according to any one of the embodiments of the present invention is a neuroendocrine neoplasm with positive PD-L1 expression and/or a high tumor mutation burden (TMB). More specifically, the neuroendocrine neoplasm suitable for use in the method, use and pharmaceutical composition according to any one of the embodiments of the present invention is a neuroendocrine neoplasm with PD-L1 expression ≥ 1% and/or a TMB ≥ 9.9 mutations/million base pairs. More preferably, the neuroendocrine neoplasm suitable for use in the method, use and pharmaceutical composition according to any one of the embodiments of the present invention is a neuroendocrine neoplasm with PD-L1 expression ≥ 10% and/or a TMB ≥ 9.9 mutations/million base pairs.

The term "tumor mutation burden (TMB)" used herein refers to the total number of gene coding errors, base substitutions, gene insertion or deletion errors detected in a somatic cell per million bases. In some embodiments of the present invention, tumor mutation burden (TMB) is estimated by analysis of somatic mutations, including coding base substitutions and the megabase insertions of the panel sequences studied. In the present invention, when a subject has a tumor mutation burden (TMB) of ≥ 9.9 mutations/million base pairs, it is predicted that the therapeutic effect of an anti-PD-1 antibody being administered alone to such a subject would be better than being administered alone to those with TMB < 9.9 mutations/million base pairs.

The method for treating a neuroendocrine neoplasm of the present invention comprises administering to an individual in need a therapeutically effective amount of an anti-PD-1 antibody. The anti-PD-1 antibody may be as described in any one of the embodiments herein, more preferably an antibody comprising light chain CDRs with amino acid sequences set forth in SEQ ID NOs: 1, 2 and 3 and heavy chain CDRs with amino acid sequences set forth in SEQ ID NOs: 4, 5 and 6, even more preferably a monoclonal antibody comprising a light chain variable region with an amino acid sequence set forth in SEQ ID NO: 7 and a heavy chain variable region with an amino acid sequence set forth in SEQ ID NO: 8, still more preferably a monoclonal antibody comprising a light chain with an amino acid sequence set forth in SEQ ID NO: 9 and a heavy chain with an amino acid sequence set forth in SEQ ID NO: 10, yet more preferably the humanized antibodies 38, 39, 41 and 48 described in Patent No. WO2014206107, and most preferably toripalimab.

In a particularly preferred embodiment, the present invention provides a method for treating a neuroendocrine neoplasm, which comprises administering to a patient with a neuroendocrine neoplasm a therapeutically effective amount of toripalimab; preferably, the patient has positive PD-L1 expression or a tumor mutation burden (TMB) of ≥ 9.9 mutations/million base pairs in peripheral blood or tumor tissue, or has both positive PD-L1 expression and a tumor mutation burden (TMB) ≥ 9.9 mutations/million base pairs in peripheral blood or tumor tissue, or has Ki-67 of ≥ 10%. In certain embodiments, the patient with a neuroendocrine neoplasm is preferably a patient with a neuroendocrine neoplasm who has an *ARID1A* gene mutation detected in peripheral blood. In certain embodiments, the patient with a neuroendocrine neoplasm is preferably a patient with a neuroendocrine neoplasm who has a chromosomal gene rearrangement detected in tumor tissue. In one embodiment, the present invention relates to a method for treating a neuroendocrine neoplasm in an effective individual or an individual with a neuroendocrine neoplasm, which comprises administering to the individual an effective amount of a standard therapy for treating a neuroendocrine neoplasm disclosed elsewhere herein.

The therapeutic agent described herein can constitute a pharmaceutical composition, such as a pharmaceutical composition comprising the anti-PD-1 antibody described herein or/and an additional anti-cancer agent other than the anti-PD-1 antibody, and an additional pharmaceutically acceptable carrier. As used herein, a "pharmaceutically acceptable carrier" includes any and all solvents, dispersion media, coatings, antibacterial and antifungal agents, isotonic and absorption delaying agents, and the like that are physiologically compatible. Preferably, the carrier suitable for use in the composition comprising the anti-PD-1 antibody is suitable for intravenous, intramuscular, subcutaneous, parenteral, spinal, or epidermal administration, such as by injection or infusion. The pharmaceutical composition of the present invention can contain one or more pharmaceutically acceptable salts, antioxidants, water, nonaqueous carriers, and/or adjuvants such as preserving agent, wetting agent, emulsifying agent, and dispersing agent.

The dosing regimen is adjusted to provide the optimal desired response, such as the maximum therapeutic response and/or the minimum adverse effect. The dose of the anti-PD-1 antibody, when administered in combination with another anti-cancer agent, can range from about 0.01 mg/kg body weight to about 20 mg/kg body weight, about 0.1 mg/kg body weight to about 10 mg/kg body weight, or can be a fixed dose of 120 mg or 240 mg. For example, the dose can be about 0.1 mg/kg body weight, about 0.3 mg/kg body weight, about 1 mg/kg body weight, about 2 mg/kg body weight, about 3 mg/kg body weight, about 5 mg/kg body weight, or about 10 mg/kg body weight. The dosing regimen is generally designed to achieve an exposure that results in sustained receptor occupancy (RO) based on the typical pharmacokinetics of Ab. A representative dosing regimen may be about once a week, about once every two weeks, about once every three weeks, about once every four weeks, about once a month, or longer. In some embodiments, the anti-PD-1 antibody is administered to an individual about once every two weeks.

### Use

The present invention also comprises an anti-PD-1 antibody or an antigen-binding fragment thereof for use in the treatment of a patient with a neuroendocrine neoplasm, and use of an anti-PD-1 antibody or an antigen-binding fragment thereof in the preparation of a medicament for treating a patient with a neuroendocrine neoplasm. The neuroendocrine neoplasm may be as described in any one of the embodiments above; preferably, the neuroendocrine neoplasm is a neuroendocrine neoplasm with a proliferation index Ki-67 ≥ 10%, and/or a neuroendocrine neoplasm with positive PD-L1 expression (≥1%) as shown in tumor tissue section analysis results and/or a neuroendocrine neoplasm with a high tumor mutation burden (TMB-H). Preferably, the neuroendocrine neoplasm is a neuroendocrine neoplasm with PD-L1 expression ≥ 10% and/or a TMB ≥ 9.9 mutations/million base pairs.

The anti-PD-1 antibody for use in the treatment of a patient with cancer may be preferably as described in any one of the embodiments herein, more preferably an antibody comprising light chain CDRs with amino acid sequences set forth in SEQ ID NOs: 1, 2 and 3 and heavy chain CDRs with amino acid sequences set forth in SEQ ID NOs: 4, 5 and 6, even more preferably a monoclonal antibody comprising a light chain variable region with an amino acid sequence set forth in SEQ ID NO: 7 and a heavy chain variable region with an amino acid sequence set forth in SEQ ID NO: 8, still more preferably a monoclonal antibody comprising a light chain with an amino acid sequence set forth in SEQ ID NO: 9 and a heavy chain with an amino acid sequence set forth in SEQ ID NO: 10, yet more preferably the humanized antibodies 38, 39, 41 and 48 described in Patent No. WO2014206107, and most preferably toripalimab.

### Method for Predicting Therapeutic Effect of Anti-PD-1 Antibody on Cancer

The method for predicting the therapeutic effect of an anti-PD-1 antibody, particularly toripalimab on cancer of an individual described herein comprises detecting a biomarker in peripheral blood of the individual prior to treatment or a chromosomal gene rearrangement in tumor tissue of the individual prior to treatment, wherein the biomarker includes, but is not limited to an *ARID1A* gene mutation.

"Gene mutation", "genomic mutation", "gene alteration" or "genomic alteration" described herein includes gene truncation, gene rearrangement/fusion, gene amplification, gene deletion, and gene substitution/insertion, etc.

The term "gene amplification" used herein refers to a process in which the copy number of a gene encoded by a specific protein is increased selectively while the number of the other genes is not increased proportionally. Under natural conditions, the gene amplification is achieved by excising repeated sequences of a gene from the chromosome and then performing extrachromosomal replication in a plasmid, or by transcribing all the repeated sequences of ribosomal RNA to give RNA transcripts and then transcribing them to give additional copies of the original DNA molecule. In the present invention, gene sequencing analysis is disclosed in some examples.

The term "gene rearrangement" used herein refers to a means of initiating transcription by transferring a gene from a location remote from a promoter to a location close to the promoter. The mechanism of gene rearrangement is a repair process of DNA double-strand breaks, in which a complex conversional transfer of repeat units occurs within or between alleles. The present invention also comprises a method for predicting the therapeutic effect of an anti-PD-1 antibody on an individual with a neoplasm by detecting the presence or absence of an *ARID1A* gene mutation. Preferably, the presence of an *ARID1A* gene mutation indicates that the individual with a neoplasm is suitable for treatment with an anti-PD-1 antibody. Preferably, the individual with a neoplasm is an individual with a neuroendocrine neoplasm. In some embodiments, the *ARID1A* gene mutation is a gene truncation.

The present invention also comprises a method for predicting the therapeutic effect of an anti-PD-1 antibody on an individual with a neoplasm by detecting the presence or absence of a chromosomal gene rearrangement. Preferably, the presence of a chromosomal gene rearrangement indicates that the individual with a neoplasm is suitable for treatment with an anti-PD-1 antibody. Preferably, the individual with a neoplasm is an individual with a solid tumor; further preferably, the individual with a neoplasm is an individual with a neuroendocrine neoplasm, more preferably, an individual with a neuroendocrine neoplasm with Ki ≥ 10%.

The present invention also comprises use of an agent for detecting an *ARID1A* gene mutation in the preparation of a kit for predicting the therapeutic effect of an anti-PD-1 antibody on cancer. Such agents include, but are not limited to, those conventionally used in assays, including but not limited to, primers, probes, agents required for PCR, etc.

The present invention also comprises use of an agent for detecting a chromosomal gene rearrangement in the preparation of a kit for predicting the therapeutic effect of an anti-PD-1 antibody on cancer. Such agents include, but are not limited to, those conventionally used in assays, including but not limited to, primers, probes, agents required for PCR, etc.

### Abbreviation

The following abbreviations are used throughout the description and examples of the present invention:
- BID: One dose, twice a day
- CDR: Complementarity determining region
- DFS: Disease-free survival
- FR: Framework region
- IgG: Immunoglobulin G
- IHC: Immunohistochemistry
- OR: Overall response
- ORR: Objective response rate
- OS: Overall survival
- PD: Progressive disease
- PFS: Progression-free survival
- PR: Partial response
- CR: Complete response
- SD: Stable disease
- DCR: Disease control rate
- DLT: Dose-limiting toxicity
- MTD: Maximum tolerated dose
- AE: Adverse event
- Q2W: One dose every 2 weeks
- QD: One dose everyday
- CSD: Chronic sun-damaged
- non-CSD: Non-chronic sun-damaged
- IRC: Independent review committee
- TRAE: Treatment-related adverse event
- SAE: Serious adverse event
- RO: Receptor occupancy
- UC: Urothelial carcinoma
- RCC: Renal cell carcinoma
- MM: Metastatic melanoma
- GEP(s): Gene expression profile
- RECIST: Response Evaluation Criteria in Solid Tumor
- irRECIST: Immune-Related Response Evaluation Criteria in Solid Tumor

The present invention is further illustrated by the following examples, which should not be construed as limiting the present invention. The contents of all references cited throughout the present application are explicitly incorporated herein by reference.

### Examples

### Example 1. Clinical Study on Anti-PD-1 Antibody for Treating Neuroendocrine Neoplasm

Inclusion criteria: eligible subjects must (1) be 18 years old or older, (2) have a locally advanced or metastatic neuroendocrine neoplasm with a Ki-67 ≥ 10%, (3) have failed to respond to a standard therapy, and (4) have an ECOG score of 0 or 1.

Subjects must have an evaluable lesion according to RECIST v1.1 criteria, and exclusion criteria are: having a history of autoimmune diseases; being diagnosed with immunodeficiency; having received a systemic steroid therapy for the past 4 weeks; having been treated with myelostimulating factor for the past 2 weeks; having received a monoclonal antibody therapy, chemotherapy or targeted small molecule therapy for the past 4 weeks; having received a radiotherapy for the past 4 weeks; having interstitial lung disease; having a history of HIV infection; having known active hepatitis B or C virus infection; having a history of tuberculosis; having other known progressive malignancies and active brain metastases or cancerous meningitis; or having received a previous immune checkpoint blockade therapy.

From Apr. 20, 2017 to Dec. 11, 2018, 53 patients with recurrent or metastatic NENs were screened, and 40 patients were enrolled and pathologically typed. Of them, 8 patients had moderately and well-differentiated neuroendocrine tumors (WD-NETs) (5 cases for grade 2, and 3 cases for grade 3) and 32 patients had poorly differentiated neuroendocrine carcinomas (PD-NECs) (grade 3). The primary sites were pancreas (9 cases) and extra-pancreatic sites (31 cases), including colorectum (10 cases), stomach (6 cases), duodenum (4 cases), esophagus (3 cases) and others (8 cases). Most of the subjects (67.5%) had liver metastases, while 17.5% of the subjects have received more than 3 systemic therapies. Demographics of the enrolled subjects are shown in Table 1.

**Table 1: Demographics of the enrolled subjects**

| Characteristics (n[%]) | | WD-NET | PD-NEC | Total |
|---|---|---|---|---|
| | | N=8 n (%) | N=32 n (%) | N=40 n (%) |
| Age | Mean value (range) (age) | 57(47-71) | 58(38-77) | 58(38-77) |
| Gender | Male | 4(50.0) | 21(65.6) | 25(62.5) |
| | Female | 4(50.0) | 11(34.4) | 15(37.5) |
| Major site | Pancreas | 4(50) | 5(15.6) | 9(22.5) |
| | Gastrointestinal tract | 3(37.5) | 20(62.5) | 23(57.5) |
| | Others | 1(12.5) | 7(21.9) | 8(20.0) |
| Liver metastasis | | 6(75.0) | 21(65.6) | 27(67.5) |
| PD-L1 expression | ≥1% | 3(37.5) | 11(34.4) | 14(35.0) |
| | <1% | 4(50.0) | 20(62.5) | 24(60.0) |
| | ≥10% | 3(37.5) | 7(21.9) | 10(25.0) |
| | <10% | 4(50.0) | 24(75.0) | 28(70.0) |
| | Unknown | 1(12.5) | 1(3.1) | 2(5.0) |
| Priority therapy | 1 | 4(50.0) | 22(68.8) | 26(65.0) |
| | 2 | 1(12.5) | 6(18.7) | 7(17.5) |
| | ≥3 | 3(37.5) | 4(12.5) | 7(17.5) |

| | | | | |
|---|---|---|---|---|
| WD-NET: well-differentiated neuroendocrine tumor; PD-NEC: poorly differentiated neuroendocrine carcinoma | | | | |

### Test drug: the anti-PD-1 antibody toripalimab (WO2014206107).

Subjects were administered with toripalimab at 3 mg/kg intravenously once every two weeks (Q2W) until the confirmation of progressive disease, intolerable toxicity, withdrawal of individual consent, investigator's decision to discontinue treatment, or until the end of treatment of 24 months. The clinical responses were evaluated once every 8 weeks for the first 6 months, and once every 12 weeks thereafter according to RECIST v1 .1 and the Immune-Related Response Evaluation Criteria in Solid Tumor (irRECIST). The clinical responses were evaluated once every 3 months after the interruption.

### Study design:

This is a phase Ib clinical trial. This study was conducted to evaluate the safety and efficacy of an anti-PD-1 antibody in treating an individual with a recurrent or metastatic neuroendocrine neoplasm.

### 1.1 Safety study

As of Aug. 30, 2019, 8.4 months after enrollment of the last subject, 40 individuals received 1 to 47 doses of toripalimab. Thirty-eight (95.0%) of 40 individuals experienced treatment-related adverse events (TRAEs). The most common TRAEs (> 20%) include proteinuria, elevated AST, hyperglycemia, elevated ALT, elevated direct bilirubin, elevated lipase, pruritus, increased creatine kinase, anemia, and fatigue, with no treatment-related deaths. The specific results are shown in Table 2.

**Table 2. Summary of treatment-related adverse events (TRAEs)**

| **N(%)** | **All** | **Grade 1** | **Grade 2** | **Grade 3** | **Grade 4** | **Grade 5** |
|---|---|---|---|---|---|---|
| **All adverse events** | **38(95.0)** | **12(30.0)** | **15(37.5)** | **10(25.0)** | **1(2.5)** | **0** |
| Proteinuria | 16(40.0) | 14(35.0) | 2(5.0) | 0 | 0 | 0 |
| Elevated AST | 15(37.5) | 12(30.0) | 2(5.0) | 1(2.5) | 0 | 0 |
| Hyperglycemia | 14(35.0) | 6(15.0) | 5(12.5) | 3(7.5) | 0 | 0 |
| Elevated ALT | 13(32.5) | 10(25.0) | 2(5.0) | 0 | 1(2.5) | 0 |
| Elevated direct bilirubin | 12(30.0) | 9(22.5) | 1(2.5) | 2(5.0) | 0 | 0 |
| Elevated lipase | 11(27.5) | 4(10.0) | 1(2.5) | 6(15.0) | 0 | 0 |
| Pruritus | 10(25.0) | 10(25.0) | 0 | 0 | 0 | 0 |
| Increased creatine kinase | 10(25.0) | 8(20.0) | 1(2.5) | 1(2.5) | 0 | 0 |
| Anemia | 9(22.5) | 7(17.5) | 2(5.0) | 0 | 0 | 0 |
| Fatigue | 8(20.0) | 8(20.0) | 0 | 0 | 0 | 0 |
| Increased amylase | 7(17.5) | 4(10.0) | 3(7.5) | 0 | 0 | 0 |
| Rash | 7(17.5) | 7(17.5) | 0 | 0 | 0 | 0 |
| Nausea | 6(15.0) | 6(15.0) | 0 | 0 | 0 | 0 |
| Leukopenia | 6(15.0) | 5(12.5) | 1(2.5) | 0 | 0 | 0 |
| Hyponatremia | 6(15.0) | 6(15.0) | 0 | 0 | 0 | 0 |
| Fever | 5(12.5) | 4(10.0) | 1(2.5) | 0 | 0 | 0 |
| Hematuresis | 5(12.5) | 5(12.5) | 0 | 0 | 0 | 0 |
| Diarrhea | 5(12.5) | 4(10.0) | 1(2.5) | 0 | 0 | 0 |
| Elevated blood uric acid | 5(12.5) | 5(12.5) | 0 | 0 | 0 | 0 |
| Neutropenia | 4(10.0) | 4(10.0) | 0 | 0 | 0 | 0 |
| Hearing loss | 4(10.0) | 4(10.0) | 0 | 0 | 0 | 0 |
| Leukocytosis | 4(10.0) | 4(10.0) | 0 | 0 | 0 | 0 |
| Hypochloremia | 4(10.0) | 4(10.0) | 0 | 0 | 0 | 0 |
| Elevated total bilirubin | 4(10.0) | 3(7.5) | 1(2.5) | 0 | 0 | 0 |
| Anorexia | 4(10.0) | 4(10.0) | 0 | 0 | 0 | 0 |
| **Immune-related AE (irAE)** | **22(55.0)** | **16(40.0)** | **4(10.0)** | **1(2.5)** | **1(2.5)** | **0** |
| Elevated AST | 15(37.5) | 12(30.0) | 2(5.0) | 1(2.5) | 0 | 0 |
| Elevated ALT | 13(32.5) | 10(25.0) | 2(5.0) | 0 | 1(2.5) | 0 |
| Elevated direct bilirubin | 12(30.0) | 9(22.5) | 1(2.5) | 2(5.0) | 0 | 0 |
| Hypothyroidism | 3(7.5) | 1(2.5) | 2(5.0) | 0 | 0 | 0 |

### 1.2 Anti-tumor activity study

The researchers evaluated the clinical responses according to RECIST v1.1 once every 8 weeks for the first 6 months and once every 12 weeks thereafter. As of Aug. 30, 2019, 29 (72.5%) subjects died, 7 (20.0%) subjects discontinued treatment due to progressive disease, 1 subject withdrew consent, and 3 subjects were still under study. The mean duration of treatment was 1.4 months (ranging from 0.5 to 21.4 months).

Of the 40 individuals evaluated, 8 cases of partial response (PR) and 6 cases of stable disease (SD) were observed with RECIST v1.1 (as shown in FIG. 1), with an ORR of 20% (95% CI 9.1-35.7) and a DCR of 35% (95% CI 20.6-51.7). Two additional cases of PR were observed with irRECIST, with an ORR of 25.0% (95% CI 12.7-41.2) and a DCR of 40% (95% CI 24.9-56.7). The median DOR was 15.2 months/RECIST v1.1, and 4 of 8 subjects still showed sustained response by the expiration date. The median PFS was 2.5 months (95% CI 1.9-3.1)/RECIST v1.1, and the median OS was 7.8 months (95% CI 5.0-10.8).

The ORRs of 18.7% and 25.0% were observed in the PD-NEC and WD-NET subgroups, respectively. Based on the tissue origin, the ORRs from extra-pancreatic GI, pancreatic NENs and non-digestible NENs were 13.0%, 22.2% and 37.5%, respectively, so that toripalimab was likely to be more effective for treatment of pancreatic NEN (22.2% ORR) and non-digestible NEN (37.5% ORR), which was higher than GI-NEN (13.1% ORR), although the difference was not statistically significant.

Two additional cases of PR were determined with the irRECIST criteria, resulting in 25.0% ORR (95% CI 12.7-41.2) and 40% DCR (95% CI 24.9-56.7). The median DOR was 15.2 months/RECIST v1.1, and 4 of 8 individuals showed sustained response before the expiration date. The median PFS was 2.5 months (95% CI 1.9-3.1)/RECIST v1.1, and the median OS was 7.8 months (95% CI 5.0-10.8), as shown in Table 3.

Based on the tissue origin, the ORRs per RECIST v1.1 from the extra-pancreatic GI, pancreatic NENs and non-digestive NENs were 13.0%, 22.2% and 37.5%, respectively.

**Table 3. Clinical effect of toripalimab in treatment of subjects with advanced NENs based on RECIST v1.1 and irRECIST criteria**

| | PD-NEC (n=32) | WD-NET (n=8) | PD-L1≥1%(n=1 4) | PD-L1 <1 % (n=24) | PD-L1≥10%(n= 10) | PD-L1 <10%(n =28) | Pancrea s (n = 9) | Extra - pancreatic GI (n = 23) | non-GI (n = 8) |
|---|---|---|---|---|---|---|---|---|---|
| RECIST | | | | | | | | | |
| PR | 6 | 2 | 6 | 2 | 5 | 3 | 2 | 3 | 3 |
| SD | 3 | 3 | 0 | 5 | 0 | 5 | 3 | 2 | 1 |
| PD | 21 | 3 | 6 | 15 | 5 | 18 | 4 | 16 | 4 |
| NE | 2 | 0 | 0 | 2 | 0 | 2 | 0 | 2 | 0 |
| ORR | 17.9% | 25.0% | 42.9% | 8.3% | 50.0% | 10.7% | 22.2% | 13.0% | 37.5% |
| DCR | 25.0% | 62.5% | 42.9% | 29.2% | 50.0% | 28.6% | 55.5% | 21.7% | 50.0% |
| irRECIST | | | | | | | | | |
| PR | 8 | 2 | 7 | 3 | 5 | 5 | 2 | 4 | 4 |
| SD | 3 | 3 | 0 | 5 | 0 | 5 | 3 | 2 | 1 |
| PD | 19 | 3 | 7 | 14 | 5 | 16 | 4 | 15 | 3 |
| NE | 2 | 0 | 0 | 2 | 0 | 2 | 0 | 2 | 0 |
| ORR | 25.0% | 25.0% | 50.0% | 12.5% | 50.0% | 17.9% | 22.2% | 17.4% | 50.0% |
| DCR | 34.4% | 62.5% | 50.0% | 33.3% | 50.0% | 35.7% | 55.5% | 26.1% | 62.5% |

| | | | | | | | | | |
|---|---|---|---|---|---|---|---|---|---|
| Note: PD-L1 positive status is defined as the presence of any tumor cells or stromal cells with membrane staining intensity ≥ 1% by SP142 IHC staining. | | | | | | | | | |

PD-NEC: poorly differentiated neuroendocrine carcinoma; WD-NET: well-differentiated neuroendocrine tumor; PR: partial response; SD: stable disease; PD: progressive disease; NE: not evaluated, 2 subjects were not evaluated after treatment; ORR: objective response rate; DCR: disease control rate; ORR = CR + PR/total number of people treated; DCR = CR + PD + SD/total number of people treated.

### 1.3 PD-L1 expression in tumors

Tumor tissue from 38 subjects was analyzed by SP142 immunohistochemical staining to obtain tumor PD-L1 expression results, of which 14 (27.5%) individuals were PD-L1⁺ (≥ 1%). The ORR after the administration of toripalimab was better in PD-L1⁺ subjects than in PD-L⁻subjects (42.9% vs. 8.3%, *p* = 0.034). The mOS was also better in PD-L1⁺ subjects than in PD-L⁻ subjects (9.1 months vs. 7.2 months), although the difference was not statistically significant. The difference in ORR was more significant in subjects with PD-L1 ≥ 10% (50.0% vs. 10.7%, *p* = 0.019) (FIG. 2, A). Subjects with PD-L1 ≥ 10% had better PFS and OS than subjects with PD-L1 < 10% (mPFS: 3.8 months vs. 2.2 months, HR = 0.50[95% C 0.24-1.06], *p* = 0.07; mOS: 9.1 months vs. 7.2 months HR= 0.55[95% C 0.24-1.23], *p* = 0.15) (FIG. 2, B and C).

### 1.4 Analysis of tumor mutation burden

Tumor biopsies and matched peripheral blood samples from 35 subjects were thoroughly sequenced by the whole exome sequencing to analyze somatic mutations within the coding regions of human genes. The median TMB value for the group of subjects with lower TMB values was 2.4 mutations (Muts)/million base pairs (Mb). Only one subject had a TMB of more than 20 mutations/million base pairs, which manifested as a partial response. Two other subjects with TMB greater than 10 mutations/million base pairs experienced PR and SD, respectively. The threshold of top 10% of TMB values (9.9 mutations/million base pairs) was selected as the TMB high threshold for this study. As shown in FIG. 2(A), the response of subjects with TMB ≥ 9.9 mutations/million base pairs (n = 4) was significantly better than those with TMB < 9.9 mutations/million base pairs (n = 34) (ORR 75.0% vs 16.1%, *p* = 0.03; DCR 100% vs 29.0%, *p* = 0.014).

The study also found that individuals with high TMB also had a clear survival advantage in PFS than individuals with low TMB, HR = 0.35 (95% CI 0.15-0.84), *p* =0.019 (as shown in FIG. 2, D). Two of the 4 subjects with high TMB had PD-L1 expression of ≥ 10%, and the two subjects showed sustained partial responses at 15.1 months and 21.9 months, respectively. Subjects with high TMB also had better mOS than those with low TMB (not reached vs. 7.5 months), HR = 0.38 (95% CI 0.13-1.12), *p* = 0.08 (FIG. 2, E).

### Example 2. Study on Correlation Between Biomarkers and Clinical Efficacy

In the experiment of Example 1, messenger RNA extracted from tumor biopsies was subjected to sequencing and expression profiling.

### Messenger RNA expression profiling

RNA was extracted from unstained fixed paraffin-embedded sections and subjected to complementary DNA synthesis and sequencing on the NovaSEquation 5000/6000 platform (Illumina, San Diego, CA). The relative abundance of each annotated transcript was expressed as transcripts per million and subjected to a log2 transformation prior to analysis. A therapeutic efficacy prediction model was established by logistic regression analysis using 12 gene panels covering inflammatory response and angiogenesis markers. Briefly, RNA transcript abundance (transcripts per million) of a selected gene was loaded into a logistic regression analysis model to obtain the best receptor operating characteristic performance with optimal fit coefficients. To obtain a single score for the signature of each sample, the average expression level of the genes containing the signature was calculated.

### Messenger RNA expression profiling in tumor biopsy

Efficient whole exome sequencing results were obtained from 35 individuals of Example 1. The most common genomic alterations include *TP53* (69%), *RB1* (37%), *CTNNB1* (20%), *CDKN2A* (14%), *KMT2D* (14%), *ARID1A* (11%), *CIC* (11%) and *TERT* (11%) (FIG. 3, A).

NENs of different organ origins showed significant genomic alterations. It was found in this study that the *MEN1* mutation (37.5%) and *DAXX* mutation (25%) were found in pancreatic NENs, while the *SMAD4* mutation (15.8%), *PTEN* mutation (15.8%) and *TERT* amplification (15.8%) were enriched in gastrointestinal NENs (FIG. 3, A). Genomic mutations of *ARID1A* were associated with clinical benefits, and 3/8 (37.5%) responders had an *ARID1A* mutation, while only 1/27 non-responders had an *ARID1A* mutation (*p* = 0.03) (FIG. 3, B).

It was also found in this study that 2 subjects were identified as microsatellite instability (MSI-H) with TMB values of 25.2 mutations/million base pairs and 8.3 mutations/million base pairs, respectively, and that the two subjects experienced PR and progressive disease, respectively. It was also found in this study that both MSI-H individuals had an *ARID1A* mutation.

In addition, it was also found in this study that a subject with sustained response (PFS 15.8+ months) had low TMB expression (1.0 mutation/million base pairs) and negative PD-L1 expression, and messenger RNA expression analysis showed that the subject had particularly high chromosomal gene rearrangements.

## Claims

1. Use of an anti-PD-1 antibody or an antigen-binding fragment thereof in the preparation of a medicament for treating a patient with a neuroendocrine neoplasm.

2. The use according to claim 1, wherein the neuroendocrine neoplasm has a proliferation index ki-67 of ≥ 10%; preferably, the neuroendocrine neoplasm is a poorly differentiated neuroendocrine carcinoma (NEC) with ki-67 ≥ 10% or a well-differentiated neuroendocrine tumor (NET) with ki-67 ≥ 10%.

3. The use according to claim 2, wherein the neuroendocrine neoplasm is a neuroendocrine neoplasm with PD-L1 ≥ 1% in a tumor tissue section by immunohistochemical staining analysis, preferably, a neuroendocrine neoplasm with PD-L1 ≥ 10% in a tumor tissue section by immunohistochemical staining analysis.

4. The use according to claim 2, wherein the neuroendocrine neoplasm is a neuroendocrine neoplasm with a high tumor mutation burden (TMB), preferably, a neuroendocrine neoplasm with a tumor mutation burden ≥ 9.9 mutations/million base pairs.

5. The use according to any one of claims 1 to 4, wherein the anti-PD-1 antibody comprises light chain complementarity determining regions (LCDRs) and heavy chain complementarity determining regions (HCDRs), wherein the LCDRs comprise amino acid sequences set forth in SEQ ID NOs: 1, 2 and 3, and the HCDRs comprise amino acid sequences set forth in SEQ ID NOs: 4, 5 and 6.

6. The use according to claim 5, wherein the anti-PD-1 antibody comprises a light chain variable region (VL) and a heavy chain variable region (VH), wherein the VL comprises an amino acid sequence set forth in SEQ ID NO: 7, and the VH comprises an amino acid sequence set forth in SEQ ID NO: 8.

7. The use according to claim 5, wherein the anti-PD-1 antibody is an anti-PD-1 antibody comprising a light chain and a heavy chain, wherein the light chain comprises an amino acid sequence set forth in SEQ ID NO: 9, and the heavy chain comprises an amino acid sequence set forth in SEQ ID NO: 10.

8. The use according to claim 5, wherein the anti-PD-1 antibody is selected from one or more of nivolumab, pembrolizumab, toripalimab, sintilimab, camrelizumab, tislelizumab and cemiplimab, preferably toripalimab.

9. The use according to claim 5, wherein the anti-PD-1 antibody is a monoclonal antibody or an antigen-binding fragment thereof.

10. The use according to any one of claims 1 to 9, wherein the anti-PD-1 antibody or the antigen-binding fragment thereof is administered at a single dose of about 0.1 mg/kg body weight to about 10.0 mg/kg body weight, e.g., about 0.1 mg/kg body weight, about 0.3 mg/kg body weight, about 1 mg/kg body weight, about 2 mg/kg body weight, about 3 mg/kg body weight, about 5 mg/kg body weight or 10 mg/kg body weight, or selected from a fixed dose of about 120 mg to about 480 mg, e.g., a fixed dose of about 120 mg, 240 mg, 360 mg or 480 mg.

11. The use according to claim 10, wherein the anti-PD-1 antibody or the antigen-binding fragment thereof is administered at a frequency of about once every week, once every two weeks, once every three weeks, once every four weeks or once a month, preferably once every two weeks.

12. The use according to claim 11, wherein the anti-PD-1 antibody or the antigen-binding fragment thereof is administered at a single dose of 1 mg/kg body weight, 3 mg/kg body weight or 10 mg/kg body weight, or of a fixed dose of 240 mg or 480 mg once every two weeks.

13. The use according to claim 10, wherein the anti-PD-1 antibody or the antigen-binding fragment thereof is administered parenterally, e.g., by intravenous infusion, in a liquid dosage form, e.g., an injection.

14. The use according to claim 10, wherein the anti-PD-1 antibody or the antigen-binding fragment thereof can be administered in cycles of one week, two weeks, three weeks, one month, two months, three months, four months, five months, half a year or longer; optionally, the cycles each can be identical or different, and at identical or different intervals.

15. Use of an agent for detecting an *ARID1A* gene mutation in peripheral blood or tumor tissue of an individual in the preparation of a kit for predicting the therapeutic effect of an anti-PD-1 antibody on a neuroendocrine neoplasm, wherein preferably, the *ARID1A* gene mutation is a gene truncation.

16. Use of an agent for detecting a chromosomal gene rearrangement in tumor tissue of an individual in the preparation of a kit for predicting the therapeutic effect of an anti-PD-1 antibody on cancer, wherein preferably, the cancer is a solid tumor; and more preferably, the cancer is a neuroendocrine neoplasm.

17. A detection kit comprising an agent for detecting an *ARID1A* gene mutation in peripheral blood or tumor tissue of an individual.

18. A detection kit comprising an agent for detecting a chromosomal gene rearrangement in tumor tissue of an individual.
